# EUROPEAN PATENT SPECIFICATION

(11) **EP 2 187 987 B1**
(45) Date of publication and mention of the grant of the patent: **09.07.2014**
(21) Application number: 08807166.7
(22) Date of filing: 26.03.2008
(51) Int. Cl.: A61F 2/91, A61F 2/915, A61F 2/82, A61L 31/02, A61L 31/04, A61L 31/14, A61L 31/16

(54) **HYBRID STENT HAVING A FIBER OR WIRE BACKBONE**
HYBRIDER STENT MIT EINEM FASER- ODER DRAHTRÜCKGRAT
STENT HYBRIDE AYANT UN SQUELETTE DE FIBRE OU DE FIL

(30) Priority: 28.03.2007 US 729516
(43) Date of publication of application: 26.05.2010
(60) Divisional application: 10004585.5
(73) Proprietor: Medinol. LTD., 91450 Jerusalem (IL)
(72) Inventor: RICHTER, Jacob, 47226 Ramat Hasharon (IL)
(74) Representative: Kuhnen & Wacker
(86) International application number: PCT/IB2008/002515
(87) International publication number: WO 2009/007850

(56) References cited:
- US-A1- 2003 212 449
- US-A1- 2006 122 691

## Description

### FIELD OF USE

The invention relates generally to stents, which are endoprostheses implanted into vessels within the body, such as a blood vessels, to support and hold open a lumen, or to secure and support other endoprostheses in vessels.

### BACKGROUND

Various stents are known in the art. Typically stents are generally tubular in shape, and are expandable from a relatively small, unexpanded diameter to a larger, expanded diameter generally to match the lumen diameter. For implantation, the stent is typically mounted at or near the end of a catheter, with the stent being held on the catheter at its relatively small, unexpanded diameter. Using a catheter, the unexpanded stent is directed through the lumen to the intended implantation site. Once the stent is at the intended implantation site, it is expanded, typically either by an internal force, for example by inflating a balloon on the inside of the stent, or by allowing the stent to self-expand, for example by removing a sleeve from around a self-expanding stent, allowing the stent to expand outwardly. In either case, the expanded stent resists the tendency of the vessel to narrow, thereby maintaining the vessel's patency.

Some examples of patents relating to stents include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

The US 2006/0122691 A1 discloses a stent provided with a series of short pieces or sections connected together by a bioresorbable polymer. The stent sections are designed to separate or articulate with time as the polymer biodegrades. The time of separation can be controlled by the characteristics of the bioresorbable polymer to allow the stent to be buried in neo-intima. By using a tube made of a bioresorbable polymer, the continuous covering of the tubing may inhibit embolization in the first few weeks after stent implantation within the walls of a vessel and timing for removal of the tube through formulation of the bioresorbable polymer can be controlled to occur when embolization is no longer a risk. When the detachment of the stent pieces or sections occurs, they are fixedly secured within the vessel and each is able to flex with the vessel independently of the other stent segments.

The US 2003/0212449 A1 discloses an expandable hybrid stent for implantation in a body lumen, such as a coronary artery. The stent generally consists of metallic cylindrical rings used in connection with polymeric links, polymeric wire or a polymeric coil. The metallic cylindrical rings can includes series of radially expandable cylindrical rings longitudinally aligned on a common axis of the stent. The rings can be interconnected by one or more polymeric links or can be disposed over an inner member consisting of a polymeric coil or a series of polymeric wires.

One object of prior stent designs has been to insure that the stent has sufficient radial strength when it is expanded so that it can sufficiently support the lumen. Stents with high radial strength, however, often tend to have a higher longitudinal rigidity than the vessel in which it is implanted. When the stent has a higher longitudinal rigidity than the vessel in which it is implanted, increased trauma to the vessel may occur at the ends of the stent, due to stress concentrations on account of the mismatch in compliance between the stented and un-stented sections of the vessel.

There remains a need in the art for a stent design that can provide sufficient radial strength while maintaining longitudinal flexibility.

### SUMMARY OF THE INVENTION

An object of the invention is to provide a stent containing a drug that more closely matches the compliance of the vessel in which it is implanted, with relatively little or no sacrifice in radial strength, even when the stent is very long.

A stent may be provided with predetermined or specific "designated detachment" points, such that after the stent is deployed, and during the motion of the vessel, the stress applied on the stent will cause the stent to separate at these designated detachment points. When the designated detachment points are arranged completely around the circumference of the stent, creating a circumferential "designated detachment" zone, the detachment at the designated detachment points separates the stent into two or more separate sections or pieces (hereafter "sections"), each able to move with the vessel independently of one another. Because each separate section can move independently, a series of separate sections can achieve greater compliance between the stented and un-stented sections of the vessel than a longer stent product, and thereby reduce stress on the vessel wall.

One mechanism of detachment is the use of bioresorbable or biodegradable material. A bioresorbable or biodegradable material is a material that is absorbed or is degraded in the body by active or passive processes. When either type of material is referred to herein, it is meant to apply to both bioresorbable and biodegradable materials. The stent may also be comprised of a metal stent having a durable polymer covering which may be continuous or fibrous in nature and may cover all or part of the metal stent structure.

The longitudinal structure of the bioresorbable or durable polymer material may be porous or it may be formed as a tube with fenestrations or a series of fibers with spaces between them, to promote faster growth of neo-intima that will cover the stents and secure them in position before degradation of the structure (in the case of the bioresorbable material), Fenestrations may also promote better stabilization of the stent. The shape of fenestration can be made in any desired size, shape or quantity.

It will be appreciated that the separation between sections can be controlled by the characteristics of the bioresorbable or durable material. Preferably, separation occurs after the stent is buried in neo-intima and the short sections are stabilized.

One method of achieving high radial resistance but low resistance to longitudinal bending, uses a stent that has separate metal sections held together by a soft longitudinal structure made from a durable polymer material.

In another embodiment the biodegradable or durable polymer is not in the form of a covering, but rather a multiplicity of fibers or wires that serve as a longitudinal back bone connecting sections of the metallic material that makes up the stent. In addition, the fibers may be of variable diameters and may contain beads, blobs or bulges that may be made of the same material as the polymer fiber or of a different material. These beads, blobs or bulges allow for variable elution dynamics for different drugs which may coat the stent. The beads, blobs, and bulges are structurally different, and provide various surface areas that are beneficial in the elution of drugs.

### BRIEF DESCRIPTION OF THE DRAWINGS

Figure 1 shows a schematic diagram of a stent, generally in the form of a cylinder, having designated detachment zones between sections;
Figure 2 shows a schematic diagram of the stent of Figure 1 after detachment, in which the stent has separated into a series of shorter sections;
Figure 3 shows a flat layout of a stent pattern in which the components in the designated detachment zones have a cross-sectional area that is sufficiently low so that the stent will separate into its constituent sections or pieces as a result of the stress placed on the stent after implantation;
Figure 4 shows a flat layout of the stent pattern of Figure 3, after separation has occurred at the designated detachment zones; and
Figure 5 shows a flat layout of a stent pattern in which the stent has a lower number of detachment components at the designated detachment zones.
Figure 6 illustrates a side view layout of a stent as separate circumferential stent pieces embedded in a bioresorbable material.
Figure 7 illustrates a side view layout of a series of short sections embedded in a bioresorbable material.
Figure 8 illustrates a side view layout of a stent made as a series of circumferential pieces or rings embedded in a bioresorbable polymer tubing with fenestrations.
Figure 9 illustrates a photomicrograph of stent members connected by a porous polymeric structure.
Figure 10 illustrates a side view layout of a series of short sections connected by longitudinal biodegradable or non-biodegradable/durable polymer fibers or wires.
Figure 11 illustrates a side view layout of a series of short sections connected by a helical biodegradable or non-biodegradable/durable polymer fibers or wires.
Figure 12A shows polymers containing beads. Beads of about 10 micrometers integrated with polymer fibers of about 1-2 micrometers are shown.
Figure 12B shows polymers containing blobs. Blobs of wider polymer on top of a web of otherwise thin polymer fibers are shown.
Figure 12C shows standard polymer fibers with no beads, blobs or bulges.
Figure 12D shows polymers containing bulges. Bulges of foreign material inside polymer fibers are shown. The bulges may contain foreign material or the same polymer the fibers are made of, optionally with drug.

### DETAILED DESCRIPTION OF THE INVENTION

The stents of the invention are defined by any of claims 1 to 20 and contain a plurality of separate stent segments and polymer fibers interconnecting said segments, wherein said polymer fibers include a plurality of areas of larger diameter, wherein one or more areas of larger diameter contain a drug.

A stent is preferably designed such that after detachment, the ends of each section created thereby are relatively smooth, so that they do not injure the vessel wall. Also, the stent is preferably configured such that the combination of separate sections has sufficient radial strength after detachment, and results in little or no significant reduction in the stent's resistance to compression.

The stent may be designed such that detachment occurs only after a period of time following implantation, so that the stent will already be buried under neointima at the time of detachment. Thus, the separate sections remaining after detachment will be held in place by the neointima and will not move relative to the lumen, i.e., they will not "telescope" into one another, and they will not move away from one another, creating unsupported gaps.

A variety of mechanisms may be used to accomplish the detachment. For example, the stent may be provided at certain points or zones along its length with components having a cross-sectional area sufficiently low so that the sections will detach from each other preferentially under the stress placed on the stent after implantation. Alternatively or additionally, the stent may be provided with certain points or zones along its length with components and/or material that is sufficiently weaker than elsewhere in the stent so that the sections will detach preferentially under the stress placed on the stent after implantation. Alternatively or additionally, the stent may be designed such that it has a lower number of components, or struts, at the designated detachment zones, so that each such component bears more load than components elsewhere in the stent. These components are configured to separate under the increased loads they bear when the stent is repeatedly stressed after implantation.

The factors contributing to detachment may be applied individually or in combination. For example, the designated detachment struts may have low cross-sectional areas and also may be formed of weaker material, or the designated detachment zones may have a reduced number of components, with or without the components having low cross-sectional areas and/or being formed of weaker material.

A stent utilizing bioresorbable or durable material may contain separate sections or pieces that are shorter than could ordinarily function as an individual stent, because they are stabilized at the time of deployment by the longitudinal structure in which they are embedded and then retained by the neo-intimal growth. The stent may be of any desired design. The stent may be made for implanting by either balloon expansion or self expansion and made of any desired stable material.

The present invention allows the bioresorbable or durable material to be manufactured at any length. In one embodiment, the stent in the supporting structure may be manufactured as a long tube and then cut to customize the length of the implanted stent for a particular patient.

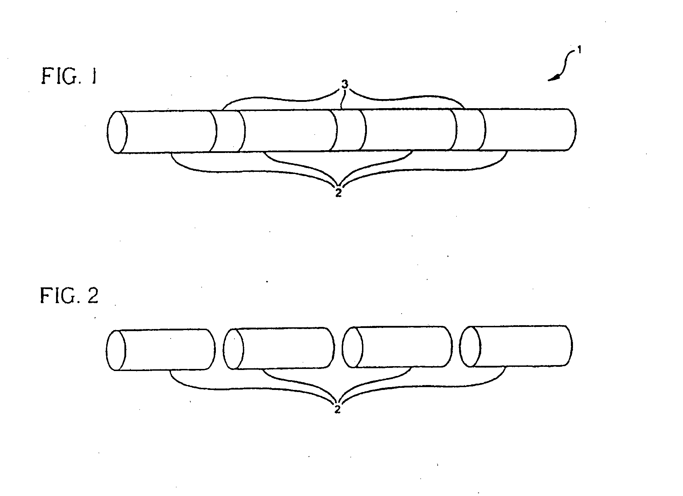

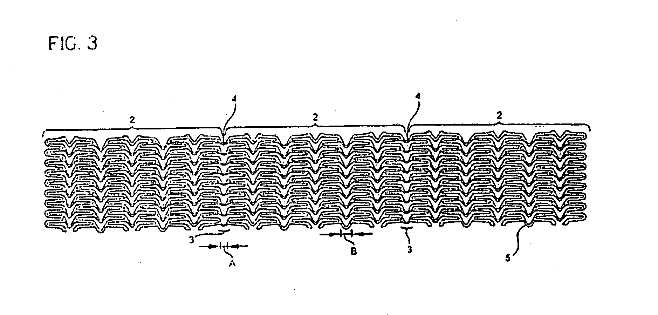

Figure 1 shows a conceptualized schematic diagram of a stent 1, generally in the form of a cylinder. The stent 1 comprises a series of separable sections 2 spaced apart by designated detachment zones 3. The designated detachment zones 3 comprise one or more designated detachment components or struts (see Figures 3 through 5).

The designated detachment zones 3 are designed such that the designated detachment components fracture or otherwise create a separation under repeated stress placed on the stent 1 after implantation. When all of the designated detachment struts around the circumference of the stent in a particular designated detachment zone 3 separate, the stent is itself separated into a series of independent sections 2, as shown in Figure 2. The designated detachment zones 3 may be designed such that detachment does not occur until some time has passed after implantation, so that the resulting separate sections 2 will already be buried under neointima at the time of detachment and therefore will not move relative to the lumen.

Persons of ordinary skill in the art will appreciate that the basic geometry of the sections 2 may take any suitable form, and that they may be formed of any suitable material. Examples of suitable structures for the sections 2 include, but are not limited to, those shown in U.S. Patent No. 5,733,303 to Israel et al., or as forming part of the NIR™ stent manufactured by Medinol Ltd. The disclosure of this patent is hereby expressly incorporated by reference into this application. Other examples of suitable structures for the sections 2, include but are not limited to, those shown in U.S. Patents Nos. 6,723,119 and 6,709,453 to Pinchasik et al., or forming part of the NIRflex™ stent, which is also manufactured by Medinol Ltd. The disclosures of these patents are also expressly incorporated by reference into this application. Other suitable stent structures may be used in the present invention and their identification is readily known to the skilled artisan based upon the teaching of the present invention.

Figure 3 shows a flat layout of a stent pattern comprising sections 2 separated by designated detachment zones 3. As here embodied, the stent pattern corresponds generally to one described in U.S. Patent No. 5,733,303, except that sections 2 are joined to each other by the designated detachment components or struts (indicated at 4) in the designated detachment zones 3.

In this embodiment, each of the designated detachment struts 4 has a reduced cross-sectional area (relative to the balance of the pattern) that is sufficiently low to allow separation at the designated detachment struts 4 under the stress placed on the stent after implantation. The amount of reduction of the cross-section of the detachment struts 4 as compared to, for example, the components labeled by reference numeral 5 in the sections 2, may be, for example, on the order of tens of percents. For example, the detachment struts 4 may be 25% to 75% thinner or narrower in the circumferential direction of the stent than the components 5.

These designated detachment struts 4 may additionally or alternatively be made of a weaker material, in order to ensure appropriate separation or fracture. The weaker material, in terms of tensile strength, may be provided either in the stock material used to form the designated detachment struts 4, or by treating the designated detachment struts 4 (or the designated detachment zones 3) after the stent has been produced, such that the treatment weakens the material of the designated detachment struts 4.

One approach for weakening the designated detachment struts is to form the entire stent of NiTi and then to treat the designated detachment struts to be Martensitic while the remaining components will be in the Austenitic phase. Another approach is to make the stent of stainless steel and hardening all but the designated detachment zones, which would be annealed.

In addition to the reduction in cross-section, the remaining geometry of the designated detachment struts may be selected to achieve the desired results. As shown in Figure 3, the width A of the row of designated detachment struts 4 may be narrower than the width of a corresponding row of components in the sections 2, for example the width B of the row of components labeled by reference numeral 5. This reduced width at the designated detachment zones 3 helps to ensure detachment at the designated detachment zones 3 under repeated longitudinal bending. Also, the designated detachment struts 4 may be made sufficiently short to reduce the length of the free ends after separation, so as not to leave long, hanging ends after detachment and thereby minimize the chance for tissue injury. For example, the length of the designated detachment struts 4 is shorter than the length of the components 5.

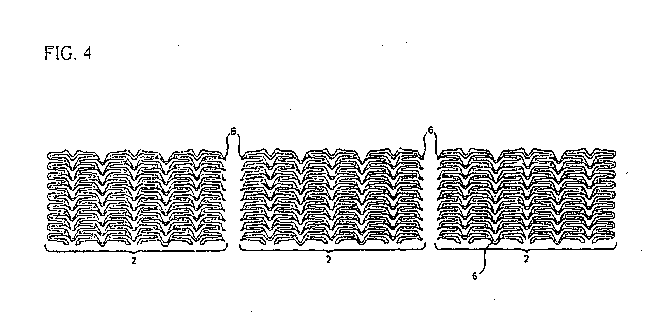

Figure 4 shows a flat layout of the stent pattern of Figure 3 after detachment has occurred at the designated detachment zones 3. As shown in Figure 4, the stent after detachment comprises a series of separated and independent sections 2. As also seen in Figure 4, because the designated detachment struts 4 were short, the length of the free ends 6 after separation is kept to a minimum.

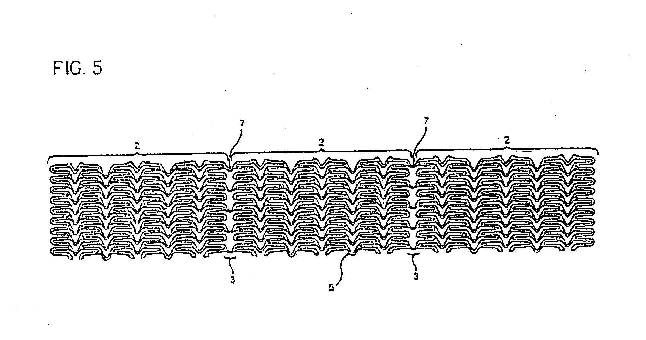

Figure 5 shows an alternative design in which the designated detachment zones 3 include fewer detachment components (here indicated at 7) around the circumference of the stent. In the embodiment shown in Figure 5, each designated detachment zone 3 has five designated detachment struts 7 around the circumference of the stent (as compared with nine in Figure 3). Of course, different numbers of designated detachment struts and stent segment components may be used, without departing from the general concept of the invention.

The designated detachment struts 7 are configured such that they detach under the loads they bear on account of the stress placed on the stent after implantation. As shown in Figure 5, the designated detachment struts 7 may also have a reduced cross-sectional area. Also, as with the designated detachment struts in other embodiments, the designated detachment struts 7 may additionally be formed of weaker material, or the designated detachment struts 7 or zones 3 may be treated to make the material weaker after production of the stent.

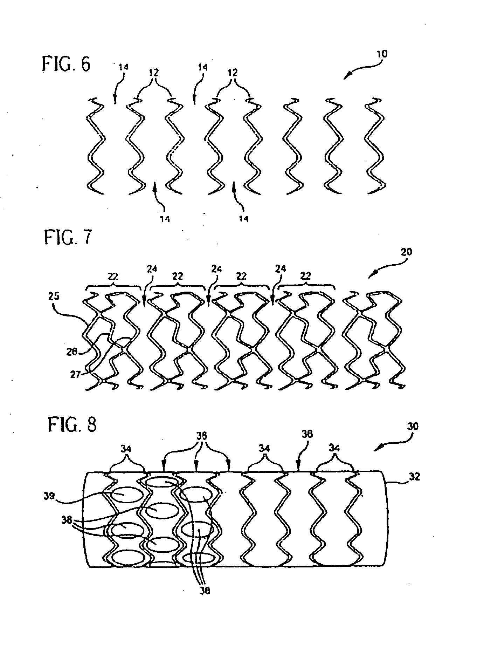

Figure 6 illustrates one example of using a bioresorbable or durable material. Stent 10 of Figure 6 comprises a series of generally circumferentially extending pieces 12 which are interconnected by a bioresorbable or durable material. This material may be placed within the spaces 14 between the pieces 12, or the latter may be embedded in the bioresorbable or durable material. Alternatively, the pieces 12 may be coated with the bioresorbable or durable material, or connected by fibers of such material or undergo any processing method known to one skilled in the art to apply the bioresorbable or durable material to the constituent pieces or sections. The polymer coating thickness or extent to how deep the pieces are embedded in the polymer may be varied and may control the timing of detachment of the constituent pieces.

Any stent design may be utilized with the bioresorbable or durable material in the manner taught by the present invention. In this example the circumferential pieces can be any structure which provides a stored length to allow radial expansion such as single sinusoidal members. However, it should be understood that the invention is not limited to any particular structure or design. For example, the circumferential pieces can be of the same design throughout the stent or they may be of different designs depending on their intended use or deployment. Thus, the invention also permits a stent design in which various circumferential pieces can have different structural or other characteristics to vary certain desired properties over the length of the stent. For example, the end pieces of the stent can be more rigid (e.g., after expansion) than those in the middle of the stent.

This example is only given as an illustration and is not meant to limit the scope of the invention. Any stent design can be used in the present invention. The individual design of each circumferential piece can be uniform or not, depending on the stent application.

One embodiment of the present invention relates to a series of otherwise separate pieces or sections which are interconnected to form a stent of a desired length by using a longitudinal structure made of bioresorbable material. The original stent structure will thus eventually disintegrate to leave a series of its constituent short sections or pieces, resulting in a longitudinal flexibility and extendibility closer to that of a native vessel. It is desirable to design the longitudinal structure such that it would promote the growth of neo-intima that will fixate the short sections or pieces into the desired position before the longitudinal structure is absorbed or degraded, and thus prevent movement of those sections thereafter.

Upon deployment in a vessel to cover a long lesion, the bioresorbable material connects the series of constituent pieces or sections together until a time when the material degrades and the constituent pieces or sections will have separated from each other. The individual sections now can articulate, move, or flex independently of each other as the vessel flexes or stretches, to allow natural movement of the vessel wall. Thus, in this embodiment of the invention, the stent bends between sections or pieces according to the natural curvature of the vessel wall.

The separation time using the bioresorbable material as the longitudinal structure of the stent can be controlled by the characteristics of the bioresorbable material. Preferably, the stent sections will have been buried in a layer of neointima and the short sections stabilized before the bioresorbable material is resorbed.

There are several advantages of using the bioresorbable material. As previously shown, there is an advantage of controlling the release of the constituent pieces or sections by modifying or choosing the characteristics of the bioresorbable material.

Additionally, the bioresorbable material does not obscure radiographs or MRI/CT scans, which allows for more accurate evaluation during the healing process. Another advantage of using the bioresorbable material is that the continuous covering provided by the bioresorbable material after the stent is deployed in a vessel is believed to inhibit or decrease the risk of embolization. Another advantage is the prevention of "stent jail" phenomenon, or the complication of tracking into side branches covered by the stent.

The depletion of the bioresorbable material covering can be controlled by modification or choosing characteristics of the bioresorbable material to allow degradation at a time about when the sections are fixated in the vessel wall and embolization is no longer a risk. Examples of altering the biodegradable or bioresorbable material by modification or changing the material characteristics of the polymer are described below as to the extent and speed a material can degrade. It should be understood that these modifications and characteristics are merely examples and are not meant to limit the invention to such embodiments.

The sections can be made of any material with desirable characteristics for balloon expandable stent or self-expandable stenting. For example, materials of this type can include but are not limited to, stainless steel, nitinol, cobalt chromium or any alloy meeting at least as a minimum the physical property characteristics that these materials exhibit.

The material of the bioresorbable material can be any material that is either readily degraded by the body and can be naturally metabolized, or can be resorbed into the body. In particular, bioresorbable materials are selected from light and porous materials which are readily colonized by living tissues to become a permanent part of the body. For example, the bioresorbable material can be, but is not limited to, a bioresorbable polymer. For example, any bioresorbable polymer can be used with the present invention, such as polyesters, polyanhydrides, polyorthoesters, polyphosphazenes, and any of their combinations in blends or as copolymers. Other usable bioresorbable polymers can include polyglycolide, polylactide, polycaprolactone, polydioxanone, poly(lactide-co-glycolide), polyhydroxybutyrate, polyhydroxyvalerate, trimethylene carbonate, and any blends and copolymers of the above polymers.

Synthetic condensation polymers, as compared to addition type polymers, are generally biodegradable to different extents depending on chain coupling. For example, the following types of polymers biodegrade to different extents (polyesters biodegrade to a greater extent than polyethers, polyethers biodegrade to a greater extent than polyamides, and polyamides biodegrade to a greater extent than polyurethanes). Morphology is also an important consideration for biodegradation. Amorphous polymers biodegrade better than crystalline polymers. Molecular weight of the polymer is also important. Generally, lower molecular weight polymers biodegrade better than higher molecular weight polymers. Also, hydrophilic polymers biodegrade faster than hydrophobic polymers. There are several different types of degradation that can occur in the environment. These include, but are not limited to, biodegradation, photodegradation, oxidation, and hydrolysis. Often, these terms are combined together and called biodegradation. However, most chemists and biologists consider the above processes to be separate and distinct. Biodegradation alone involves enzymatically promoted break down of the polymer caused by living organisms.

As a further advantage of the invention, the structure may be embedded with drug that will inhibit or decrease cell proliferation or will reduce restenosis in any way. Further, a material containing a longitudinal structure of fibers provides a continuous structure with small inter-fiber distance and provides a more uniform elution bed as a matrix for eluting drug. In one embodiment, the constituent pieces or sections may be treated to have active or passive surface components such as drugs that will be advantageous for the longer time after those sections are exposed by bioresorption of the longitudinal structure.

In an embodiment of the invention, it is desirable to use the polymer structure, whether durable or bioabsorbable, as a matrix for eluting drug to the wall of the vessel stented or into the lumen. As discussed above, the polymer may contain such drug for elution into the vessel wall. The potential problem is that some drugs are effective only when eluting in a controlled fashion over a long time, typically from 1 week to several months. One major factor of the rate of release of drugs from a polymer is the thickness of the layer the drug has to traverse on its way to the surrounding fluid. The polymer fibers that make up the longitudinal back-bone or structure of the hybrid stent may be desirable formed of a small diameter in the range of 1-5 micrometer, which may be too small a diameter to allow long enough drug release for certain applications.

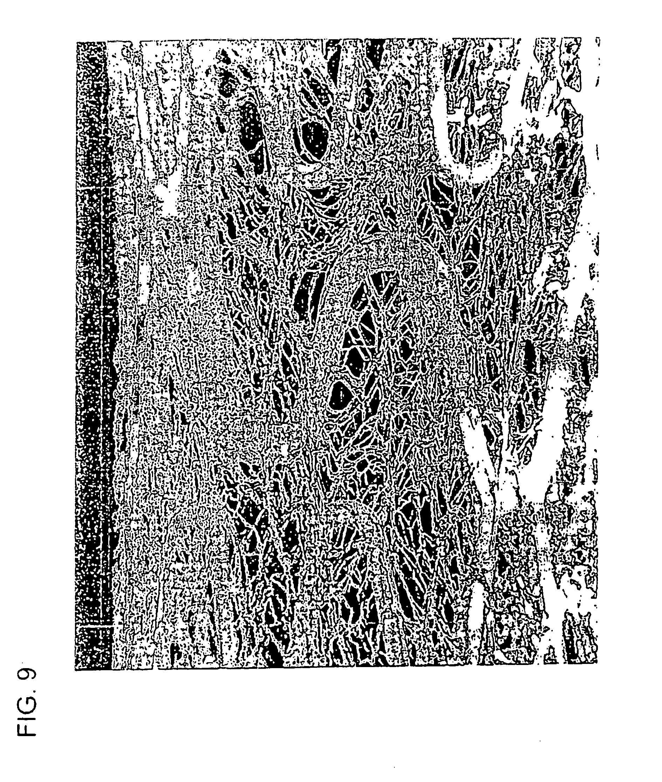

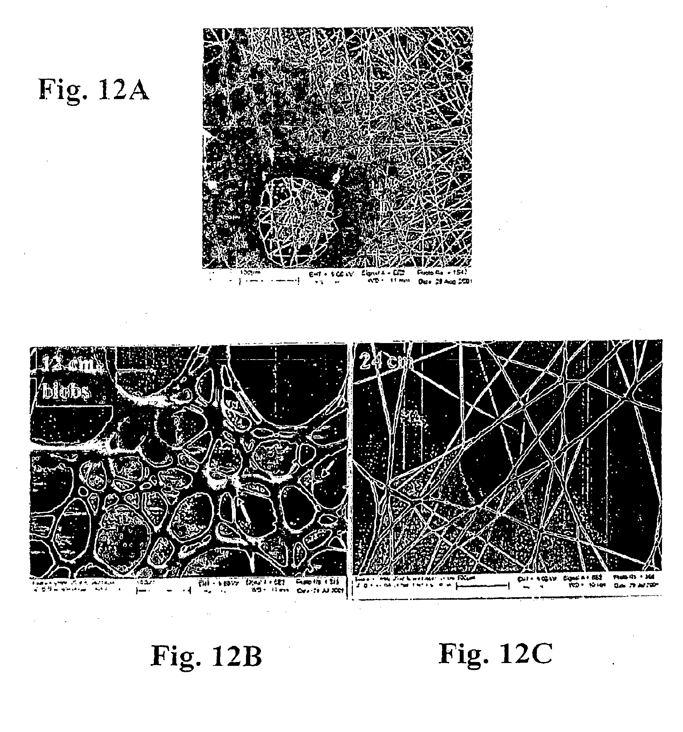

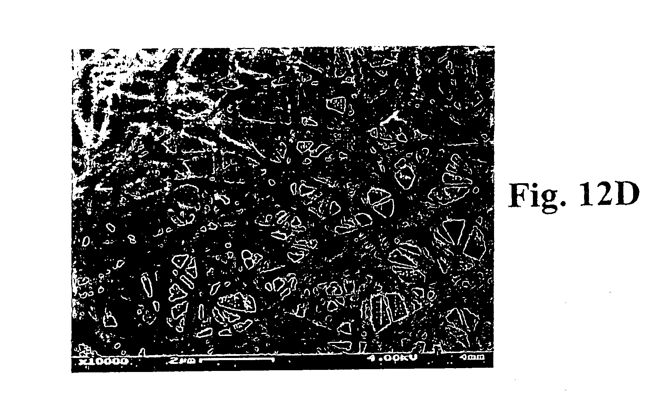

In forming polymer fibers, it may be advantageous to include in the fibers areas of much larger diameters, referred to herein as beads, blobs or bulges and described, for example with reference to Figures 12A-D. These beads, blobs or bulges can be of the same materials as the polymer fiber or they may be formed of a different material. In another aspect of the invention, the polymer fibers may include beads, blobs or bulges of polymer which may further contain one or more drugs to be released from such polymer. The typical diameter or thickness of such beads, blobs or bulges may preferably be in the range of 5-50 micrometers as necessitated by the elution dynamics of different drugs and different polymers. Figures 12A-D illustrate beads (Fig. 12A), blobs (Fig. 12B) and bulges (Fig. 12D). As shown in these figures, beads, blobs and bulges have several structural differences. Each configuration has different surface areas that are beneficial in drug elution. Figure 12C illustrates fibers which do not contain bead, blobs or bulges may also be useful in the instant invention and may, for example, be used in forming the embodiment illustrated in Figure 9. These beads, blobs or bulges may be formed of a different material, or the same polymer material and may contain one or more drugs.

Figure 7 illustrates another example of a stent 20 of the present invention. Instead of being made of a series of circumferential pieces or members as in Figure 6, this embodiment contains short sections indicated at 22. Again, as with Figure 6, these stent sections 22 can be any design and are not limited to the embodiment shown in Figure 7. Stent 20, as with the stent of Figure 6, can have identical short stent sections or not depending on the application of the stent.

The stent sections may be made of any suitable material and may form any acceptable design. The stent may be balloon expandable or self-expandable.

Example designs of stents are described in U.S. Patent No. 6,723,119, which is incorporated herein *in toto*, by reference. Another example design is the NIRflex stent which is manufactured by Medinol, Ltd. One such example is shown in Figure 7. This design criteria can result in short sections which provide longitudinal flexibility and radial support to the stented portion of the vessel. Other stent designs are readily available in the art, and can be used in the invention.

The bioresorbable material can be disposed within interstices 24 and/or embedded throughout the stent segments. The bioresorbable material may cover the entire exterior or only a portion of the stent segments or fully envelop all the segments.

Figure 8 illustrates yet another example of the present invention in the form of stent 30 having a bio-resorbable material 32 in the form of a tube. As here embodied, the tube interconnects circumferential pieces (or members) 34 with the bio-resorbable material filling interstices 36. The pieces 34 illustrated in figure 8 are single sinusoidal members, but can be of any design or multitude of designs as previous discussed.

Stent 30 may also include fenestrations 38. Fenestrations can be any shape desired and can be uniformly designed such as the formation of a porous material for example, or individually designed. The non-continuous layered material can also be formed in other ways such as a collection of bioresorbable fibers connecting the pieces. Fenestration of the bioresorbable cover may promote faster growth of neo-intima and stabilization of the short segments before integration or degradation of the bioresorbable material. The present invention allows the bioresorbable material to be manufactured at any length and then cut in any desired length for individual functioning stents to assist manufacturing the stent. For example, in the case of bioresorbable polymer tubing illustrated in Figure 8, the tubing can be extruded at any length and then cut to customize the stent, either by the manufacturer or by the user.

Figure 9 illustrates a photomicrograph of stent members or sections connected by a porous longitudinal structure along a longitudinal axis of the stent. This longitudinal structure may or may not be polymeric, depending on the properties desired. In one embodiment, the longitudinal structure is a porous fiber mesh, such as a durable polymer. One example of such a material includes, but is not limited to, polytetrafluoroethylene (ePTFE). One skilled in the art will recognized other materials having similar beneficial properties that can be used in the invention and function as durable polymer fiber. Such other materials can readily be used in the present invention. The longitudinal structure, among other functions, provides longitudinal flexibility to the stent members. The stent sections may or may not be a metallic structure, depending on the desired properties. The longitudinal structure also may provide a continuous structure having small inter-fiber distances and forming a matrix. This matrix may be used for eluting a drug and would provide a more uniform elution bed over conventional methods.

It may be advantageous to employ a light and porous polymeric material. For example, a fibrous material may be constructed so that the fibers provide a longitudinal structure thereby enhancing the overall flexibility of the stent device. Such a material may be applied to a stent or stent pieces in a continuous or non-continuous manner depending upon the particular needs of the structure contemplated. The material may be any polymeric material. An example of such a material is expanded polytetrafluoroethylene (ePTFE), but is not limited to this material. The polymeric material can form a porous fiber mesh that is a durable polymer. The longitudinal structure serves at least two functions. First, the longitudinal structure is more longitudinally flexible than a conventional metallic structures. Second, the polymeric material is a continuous structure with small inter-fiber distance and can be used as a matrix for eluting drug that would provide a more uniform elution bed.

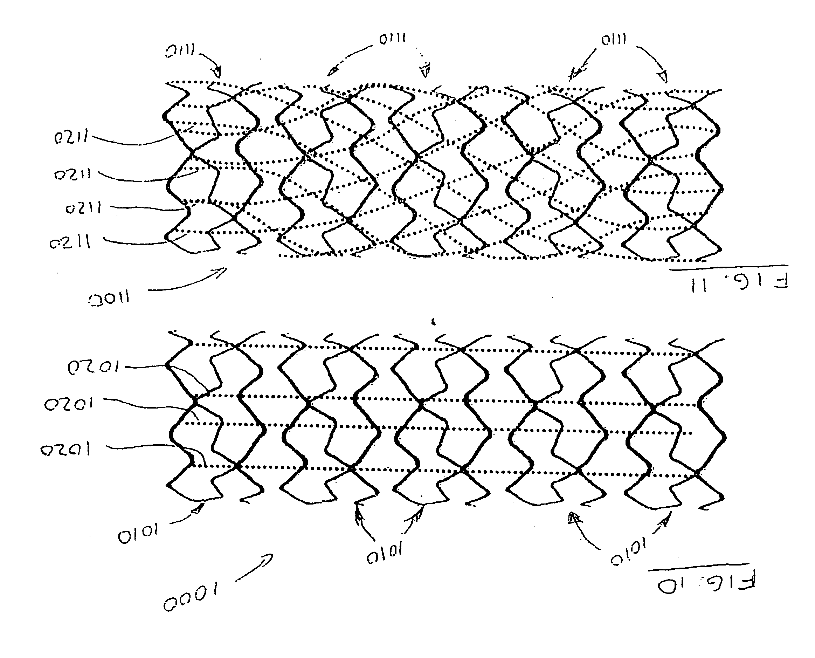

According to another aspect of the invention, one or more of the stent sections may initially be connected by biodegradable or durable polymer fibers that extend generally longitudinally of the initially assembled stent. Figure 10 illustrates a side view of a stent 1000 that has a series of short sections 1010 connected by longitudinal biodegradable or non-biodegradable/durable polymer fibers or wire-like strands 1020. In this embodiment, the fibers or wires are substantially straight although not limited to just the configuration shown. For example, the fiber or wire is oriented substantially parallel along the longitudinal axis of the stent.

In an alternate approach, the polymer fibers may generally extend in an end-to-end direction but are not necessarily parallel to the longitudinal axis of the initially constructed stent. Figure 11 illustrate a side view of a stent 1100 that has a series of short sections 1110 connected by a helical biodegradable or non-biodegradable/durable polymer fibers or wires 1120. In this embodiment the fibers or wires are helically wound around the stent segments. Again, it is within the scope of the invention to have several different configurations of these polymers or fibers to connect the stent segments. The polymers or fibers can be either biodegradable or durable.

It is within the scope of the invention to have several different configurations of these polymers or fibers to connect the stent segments. The polymers or fibers can be either biodegradable or durable. In addition, the stent design can be any stent design available. For example, the stent design can comprise a stent wherein each of said plurality of sections is formed of a single or multiple sinusoidal patterns. The stent may contain single sinusoidal patterns having different configuration from others. The stent design may also have a plurality of sections having a plurality of sinusoidal patterns. The stent may have sinusoidal patterns uniformly or non-uniformly designed and distributed throughout the stent.

Each stent section may further comprises a first loop containing section with loops occurring at a first frequency and a second loop containing section with loops also occurring at said first frequency and a third loop containing section having loops occurring at a second frequency that is higher than said first frequency. The third loop containing section may or may not be disposed between the first and second loop containing sections, and consecutively joined for at least two repetitions to said first and second loop containing sections. Stent strut width may vary depending on the embodiment.

The devices may contain one or more amorphous metal alloys. The method of heat extrusion is very flexible and many combinations of metals can be made into an amorphous metal alloy. By way of example, iron-based, cobalt-based alloys, copper-based amorphous metal alloys, as well as others may be manufactured using heat extrusion as described herein. In certain embodiments, the amorphous metal alloys may comprise a metalloid, non-limiting examples of which include silicon, boron, and phosphorus. One possible amorphous metal alloy is an Fe--Cr--B--P alloy. Many other similar alloys are suitable and known to one of ordinary skill in the art.

In certain preferred embodiments, the amorphous metal alloys contemplated by this invention exhibit significantly lower conductance or are non-conductive, compared to their crystalline or polycrystalline counterparts.

The amorphous metal alloy components of this invention may be combined or assembled with other components, either amorphous metal or otherwise, in order to form intraluminal implants. For example, the amorphous metal alloy components may be combined with a biocompatible polymer, a biodegradable polymer, a therapeutic agent (e.g., a healing promoter as described herein) or another metal or metal alloy article (having either a crystalline or amorphous microstructure). These amorphous metal alloys have many properties that make them suitable for use as implants, including high mechanical strength, resistance to fatigue, corrosion resistance, and biocompatibility.

It should be understood that the above description is only representative of illustrative examples of embodiments. For the reader's convenience, the above description has focused on a representative sample of possible embodiments, a sample that teaches the principles of the invention. Other embodiments may result from a different combination of portions of different embodiments. The description has not attempted to exhaustively enumerate all possible variations.

The invention is defined by the appended claims.

## Claims

1. A stent for implantation in a vessel, comprising:
a plurality of separate stent segments; and
polymer fibers interconnecting said segments, wherein said polymer fibers include a plurality of areas of varying diameter, wherein one or more areas of larger diameter contain a drug.

2. The stent in claim 1, wherein each of said plurality of segments is formed of a single sinusoidal pattern.

3. The stent in claim 2, wherein each of said single sinusoidal patterns is uniform.

4. The stent of claim 1, wherein each of said plurality of segments have a plurality of sinusoidal patterns.

5. The stent of claim 4, wherein each of said plurality of sinusoidal patterns are uniformly designed.

6. The stent of claim 1, having the polymer fibers extending in a plurality of directions relative to the longitudinal axis of the stent.

7. The stent of claim 1, having the polymer fibers extending in a single direction relative to the longitudinal axis of the stent.

8. The stent according to claim 1, wherein said plurality of segments separate from each other in a controlled manner in response to physiological conditions.

9. The stent of claim 8, wherein said polymer fibers inhibit embolization.

10. The stent of claim 8, wherein said polymer fibers are wires.

11. The stent of claim 8, wherein said polymer fibers are durable.

12. The stent of claim 8, wherein said stent is balloon expandable or self-expandable.

13. The stent of claim 8, wherein each segment further comprises a plurality of sinusoidal patterns, said sinusoidal patterns are generally arranged in the circumferential direction of the stent and are periodically interconnected thereto.

14. The stent of claim 1, wherein each stent segment further comprises a first loop containing section with loops occurring at a first frequency and a second loop containing section with loops also occurring at said first frequency and a third loop containing section having loops occurring at a second frequency that is higher than said first frequency, said third loop containing section being disposed between said first and second loop containing sections, and consecutively joined for at least two repetitions to said first and second loop containing sections.

15. The stent of claim 14, wherein said first and said third loop containing sections or said second and said third loop containing sections form at least one cell, and said higher frequency loops are in a ratio 3:2 to said low frequency loops.

16. The stent of claim 14, wherein said higher frequency loop containing section is smaller in width compared to said lower frequency loop containing section.

17. The stent of claim 14, wherein said higher frequency loop containing section is 180 degrees out of phase with adjacent high frequency loop containing sections.

18. The stent of claims 1 or 8, wherein the segments are made of an amorphous alloy.

19. The stent of claim 1 or 8, , wherein said polymer fibers are wires extending in a non-parallel direction to the longitudinal axis of the stent.

20. The stent of claim 19, wherein said polymer fibers or wires are helically wound.

## Patentansprüche

1. Stent zum Implantieren in ein Gefäß, aufweisend
eine Mehrzahl an separaten Stentsegmenten; und
Polymerfasem, die die Segmente miteinander verbinden, wobei die Polymerfasern eine Mehrzahl an Bereichen mit unterschiedlichem Durchmesser beinhalten, wobei einer oder mehrere Bereiche mit größerem Durchmesser ein Medikament enthalten.

2. Stent nach Anspruch 1, wobei jedes von der Mehrzahl an Segmenten aus einem einzelnen sinusförmigen Muster besteht.

3. Stent nach Anspruch 2, wobei jedes von den einzelnen sinusförmigen Mustern einheitlich ist.

4. Stent nach Anspruch 1, wobei jedes von der Mehrzahl an Segmenten eine Mehrzahl an sinusförmigen Mustern aufweist.

5. Stent nach Anspruch 4, wobei jedes von der Mehrzahl an sinusförmigen Mustern einheitlich gestaltet ist.

6. Stent nach Anspruch 1, wobei die Polymerfasern relativ zu der Längsachse des Stents in einer Mehrzahl an Richtungen verlaufen.

7. Stent nach Anspruch 1, wobei die Polymerfasern relativ zu der Längsachse des Stents in einer einzigen Richtung verlaufen.

8. Stent nach Anspruch 1, wobei die Mehrzahl an Segmenten als Reaktion auf physiologische Bedingungen kontrolliert voneinander getrennt werden.

9. Stent nach Anspruch 8, wobei die Polymerfasern die Embolisierung hemmen.

10. Stent nach Anspruch 8, wobei die Polymerfasern Drähte sind.

11. Stent nach Anspruch 8, wobei die Polymerfasern beständig sind.

12. Stent nach Anspruch 8, wobei der Stent ballonexpandierbar oder selbstexpandierbar ist.

13. Stent nach Anspruch 8, wobei jedes Segment ferner eine Mehrzahl an sinusförmigen Mustern aufweist, wobei die sinusförmigen Muster allgemein in der Umfangsrichtung des Stents angeordnet und periodisch dazu verbunden sind.

14. Stent nach Anspruch 1, wobei jedes Stentsegment ferner einen ersten Schlaufenabschnitt aufweist, bei dem Schlaufen mit einer ersten Frequenz auftreten, und einen zweiten Schlaufenabschnitt aufweist, bei dem die Schlaufen auch mit der ersten Frequenz auftreten, und einen dritten Schlaufenabschnitt aufweist, bei dem Schlaufen mit einer zweiten Frequenz auftreten, die höher ist als die erste Frequenz, wobei sich der dritte Schlaufenabschnitt zwischen den ersten und zweiten Schlaufenabschnitten befindet, und nachfolgend für mindestens zwei Wiederholungen mit dem ersten und zweiten Schlaufenabschnitt verbunden wird.

15. Stent nach Anspruch 14, wobei der erste und der dritte Schlaufenabschnitt oder der zweite und der dritte Schlaufenabschnitt zumindest eine Zelle bilden, und die Schlaufen mit höherer Frequenz in einem Verhältnis von 3:2 zu den Schlaufen mit niedriger Frequenz vorhanden sind.

16. Stent nach Anspruch 14, wobei der Schlaufenabschnitt mit höherer Frequenz in der Breite kleiner ist als der Schlaufenabschnitt mit niedrigerer Frequenz.

17. Stent nach Anspruch 14, wobei der Schlaufenabschnitt mit höherer Frequenz zu den benachbarten Schlaufenabschnitten mit hoher Frequenz um 180 Grad phasenverschoben ist.

18. Stent nach Ansprüchen 1 oder 8, wobei die Segmente aus einer amorphen Legierung bestehen.

19. Stent nach Anspruch 1 oder 8, wobei die Polymerfasern Drähte sind, die in einer zu der Längsachse des Stents nicht parallelen Richtung verlaufen.

20. Stent nach Anspruch 19, wobei die Polymerfasern oder Drähte spiralförmig gewunden sind.

## Revendications

1. Stent pour implantation dans un vaisseau, comprenant :
une pluralité de segments de stent séparés ; et
des fibres de polymère interconnectant lesdits segments, dans lequel lesdites fibres de polymère comportent une pluralité de zones de diamètre variable, dans lequel une ou plusieurs zones de diamètre plus grand contiennent un médicament.

2. Stent selon la revendication 1, dans lequel chacun de ladite pluralité de segments est constitué d'un motif sinusoïdal unique.

3. Stent selon la revendication 2, dans lequel chacun desdits motifs sinusoïdaux uniques est uniforme.

4. Stent selon la revendication 1, dans lequel chacun de ladite pluralité de segments possède une pluralité de motifs sinusoïdaux.

5. Stent selon la revendication 4, dans lequel chacun de ladite pluralité de motifs sinusoïdaux est conçu de manière uniforme.

6. Stent selon la revendication 1, dont les fibres de polymère s'étendent dans une pluralité de directions par rapport à l'axe longitudinal du stent.

7. Stent selon la revendication 1, dont les fibres de polymère s'étendent dans une direction unique par rapport à l'axe longitudinal du stent.

8. Stent selon la revendication 1, dans lequel ladite pluralité de segments se séparent les uns des autres d'une manière contrôlée en réponse aux conditions physiologiques.

9. Stent selon la revendication 8, dans lequel lesdites fibres de polymère inhibent l'embolisation.

10. Stent selon la revendication 8, dans lequel lesdites fibres de polymère sont des fils.

11. Stent selon la revendication 8, dans lequel lesdites fibres de polymère sont durables.

12. Stent selon la revendication 8, dans lequel ledit stent est dilatable par ballonnet ou auto-dilatable.

13. Stent selon la revendication 8, dans lequel chaque segment comprend en outre une pluralité de motifs sinusoïdaux, lesdits motifs sinusoïdaux sont généralement agencés dans la direction circonférentielle du stent et sont périodiquement interconnectés à celui-ci.

14. Stent selon la revendication 1, dans lequel chaque segment de stent comprend en outre une première section contenant des boucles avec des boucles survenant à une première fréquence, une deuxième section contenant des boucles avec des boucles survenant également à ladite première fréquence et une troisième section contenant des boucles ayant des boucles survenant à une seconde fréquence qui est supérieure à ladite première fréquence, ladite troisième section contenant des boucles étant disposée entre lesdites première et deuxième sections contenant des boucles, et jointe consécutivement sur au moins deux répétitions auxdites première et deuxième sections contenant des boucles.

15. Stent selon la revendication 14, dans lequel lesdites première et troisième sections contenant des boucles ou lesdites deuxième et troisième sections contenant des boucles forment au moins une cellule, et lesdites boucles de fréquence plus élevée sont présentes dans un rapport 3:2 par rapport auxdites boucles de fréquence faible.

16. Stent selon la revendication 14, dans lequel ladite section contenant des boucles de fréquence plus élevée est d'une largeur inférieure à ladite section contenant des boucles de fréquence plus faible.

17. Stent selon la revendication 14, dans lequel ladite section contenant des boucles de fréquence plus élevée est déphasée de 180 degrés par rapport aux sections contenant des boucles de fréquence élevée adjacentes.

18. Stent selon la revendication 1 ou 8, dans lequel les segments sont constitués d'un alliage amorphe.

19. Stent selon la revendication 1 ou 8, dans lequel lesdites fibres de polymère sont des fils s'étendant dans une direction non parallèle à l'axe longitudinal du stent.

20. Stent selon la revendication 19, dans lequel lesdites fibres de polymère ou lesdits fils sont enroulé(e)s de manière hélicoïdale.
